# EUROPEAN PATENT SPECIFICATION

(11) **EP 2 400 843 B1**
(45) Date of publication and mention of the grant of the patent: **11.11.2015**
(21) Application number: 10746681.5
(22) Date of filing: 22.02.2010
(51) Int. Cl.: A01N 43/40, A61K 31/445, A61P 31/14

(54) **IMINOSUGARS AND METHODS OF TREATING ARENAVIRAL INFECTIONS**
IMINOZUCKER UND VERFAHREN ZUR BEHANDLUNG VON ARENAVIRIDAE-ERKRANKUNGEN
IMINOSUCRES ET PROCÉDÉS DE TRAITEMENT D'INFECTIONS ARÉNAVIRALES

(30) Priority: 04.09.2009 US 272251 P; 24.02.2009 US 202391 P
(43) Date of publication of application: 04.01.2012
(73) Proprietor: United Therapeutics Corporation, Silver Spring, MD 20910 (US); University of Oxford, Oxford OX1 3QU (GB)
(72) Inventor: RAMSTEDT, Urban, Silver Spring, MD 20910 (US); KLOSE, Brennan, Silver Spring, MD 20910 (US); ZITZMANN, Nicole, Oxford OX1 4TL (GB); DWEK, Raymond, A., Oxford OX2 9AU (GB); BUTTERS, Terry, D., Oxford OX44 9BS (GB)
(74) Representative: Schneider, Michael
(86) International application number: PCT/US2010/024914
(87) International publication number: WO 2010/099064

(56) References cited:
- WO-A1-99/29321
- WO-A1-2006/124676
- US-A1- 2007 275 998
- SILBER A M ET AL: "The effects of oligosaccharide trimming inhibitors on glycoprotein expression and infectivity of Junin virus", FEMS MICROBIOLOGY LETTERS, BLACKWELL PUBLISHING, AMSTERDAM, NL, vol. 109, no. 1, 1 May 1993 (1993-05-01), pages 39-43, XP023853774, ISSN: 0378-1097, DOI: 10.1111/J.1574-6968.1993.TB06140.X [retrieved on 1993-05-01]
- WRIGHT K E ET AL: "POST-TRANSLATIONAL PROCESSING OF THE GLYCOPROTEINS OF LYMPHOCYTIC CHORIOMENINGITIS VIRUS", VIROLOGY, vol. 177, no. 1, 1990, pages 175-183, XP023059022, ISSN: 0042-6822
- CHANG JINHONG ET AL: "Novel Imino Sugar Derivatives Demonstrate Potent Antiviral Activity against Flaviviruses", ANTIMICROBIAL AGENTS AND CHEMOTHERAPY, AMERICAN SOCIETY FOR MICROBIOLOGY, WASHINGTON, DC, US, vol. 53, no. 4, 17 February 2009 (2009-02-17), pages 1501-1508, XP009154210, ISSN: 0066-4804, DOI: 10.1128/AAC.01457-08 [retrieved on 2009-02-17]
- BUTTERS ET AL.: 'Imino sugar inhibitors for treating the lysosomal glycosphingolipidoses.' GLYCOBIOLOGY vol. 15, no. 10, 2005, pages 43R - 52R, XP008124586
- MEHTA ANAND ET AL: "Imino sugars that are less toxic but more potent as antivirals, in vitro, compared with N-n-nonyl DNJ.", ANTIVIRAL CHEMISTRY & CHEMOTHERAPY SEP 2002, vol. 13, no. 5, September 2002 (2002-09), pages 299-304, ISSN: 0956-3202
- MARKLAND W ET AL: "Broad-spectrum antiviral activity of the IMP dehydrogenase inhibitor VX-497: a comparison with ribavirin and demonstration of antiviral additivity with alpha interferon.", ANTIMICROBIAL AGENTS AND CHEMOTHERAPY APR 2000, vol. 44, no. 4, April 2000 (2000-04), pages 859-866, ISSN: 0066-4804
- SCHUL WOUTER ET AL: "A dengue fever viremia model in mice shows reduction in viral replication and suppression of the inflammatory response after treatment with antiviral drugs.", THE JOURNAL OF INFECTIOUS DISEASES 1 MAR 2007, vol. 195, no. 5, 1 March 2007 (2007-03-01) , pages 665-674, ISSN: 0022-1899

## Description

### RELATED APPLICATIONS

The present application claims priority to U.S. provisional applications nos. 61/202,391 filed February 24, 2009 and 61/272,251 filed September 4, 2009.

### FIELD

The present application relates to iminosugars and treating viral infections with iminosugars and, in particular, to the use of iminosugars for treatment and prevention of viral infections caused by or associated with a virus belonging to the Arenaviridae family.

### SUMMARY

One embodiment is treating or preventing a disease or condition caused by or associated with a virus belonging to the Arenaviridae family, comprising administering to a subject in need thereof a compound of the formula, 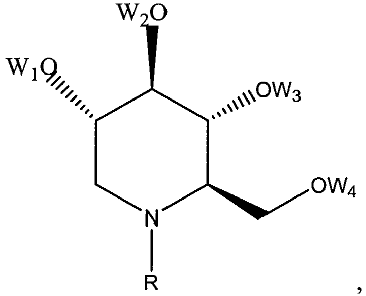 wherein R is either selected from substituted or unsubstituted alkyl groups, substituted or unsubstituted cycloalkyl groups, substituted or unsubstituted aryl groups, or substituted or unsubstituted oxaalkyl groups; or wherein R is 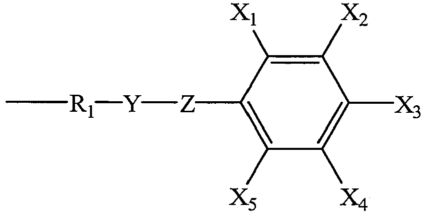 R₁ is a substituted or unsubstituted alkyl group;
X₁₋₅ are independently selected from H, NO₂, N₃, or NH₂;
Y is absent or is a substituted or unsubstituted C₁-alkyl group, other than carbonyl; and
Z is selected from a bond or NH; provided that when Z is a bond, Y is absent, and provided that when Z is NH, Y is a substituted or unsubstituted C₁-alkyl group, other than carbonyl; and
wherein W₁₋₄ are independently selected from hydrogen, substituted or unsubstituted alkyl groups, substituted or unsubstituted haloalkyl groups, substituted or unsubstituted alkanoyl groups, substituted or unsubstituted aroyl groups, or substituted or unsubstituted haloalkanoyl groups.

### DRAWINGS

Figures 1(A)-(E) present chemical formulas of the following iminosugars: A) *N*-Butyl deoxynojirimycin (NB-DNJ or UV-1); B) *N*-Nonyl deoxynojirimycin (NN-DNJ or UV-2); C) *N*-(7-Oxadecyl)deoxynojirimycin (N7-O-DNJ or UV-3); D) *N*-(9-Methoxynonyl) deoxynojirimycin (N9-DNJ or UV-4); E) *N*-(*N*-{4'-azido-2'-nitrophenyl}-6-aminohexyl)deoxynojirimycin (NAP-DNJ or UV-5).

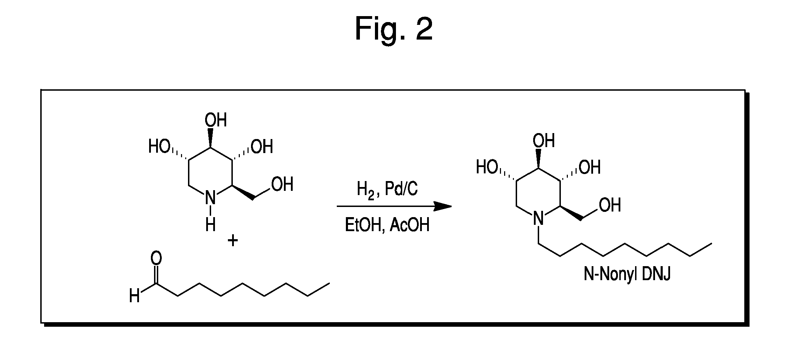

Figure 2 is a synthesis scheme for NN-DNJ.

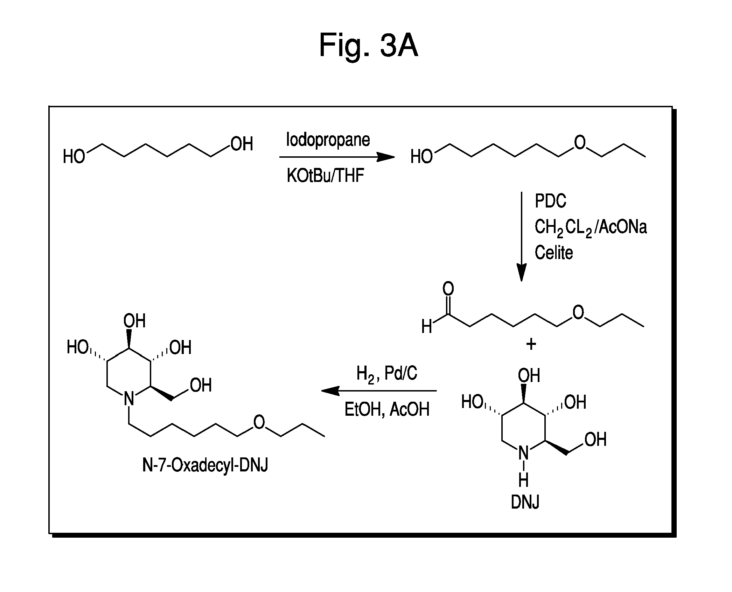

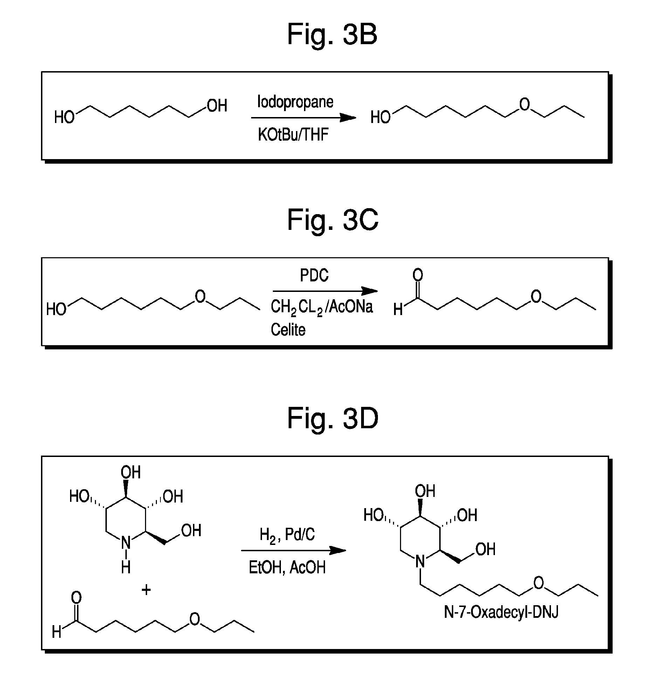

Figures 3A-D illustrate synthesis of N7-O-DNJ. In particular, Figure 3A shows a sequence of reactions leading to N7-O-DNJ; Figure 3B illustrates preparation of 6-propyloxy-1-hexanol; Figure 3C illustrates preparation of 6-propyloxy-1-hexanal; Figure 3D illustrates synthesis of N7-O-DNJ.
Figures 4A-C relate to synthesis of *N*-(9-Methoxynonyl) deoxynojirimycin. In particular,

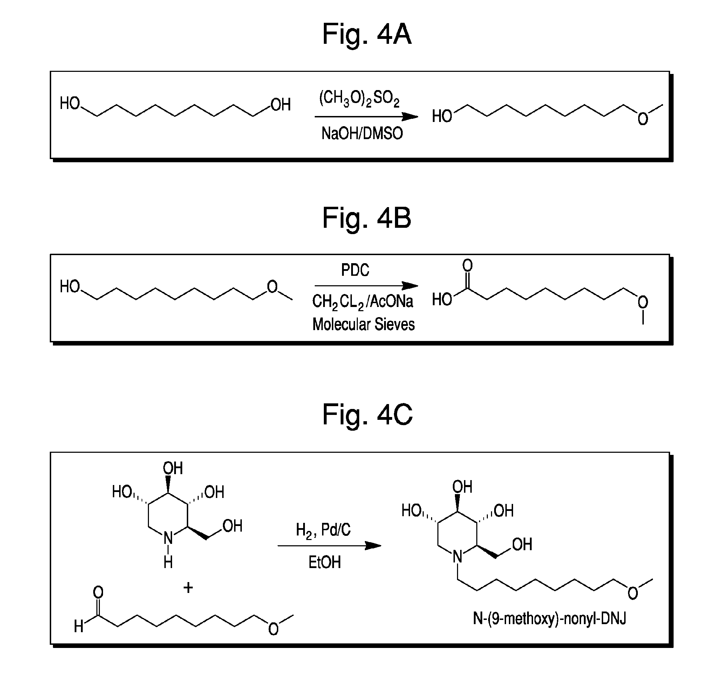

Figure 4A illustrates preparation of 9-methoxy-1-nonanol; Figure 4B illustrates preparation of 9-methoxy-1-nonanal; Figure 4C illustrates synthesis of *N*-(9-Methoxynonyl) deoxynojirimycin.

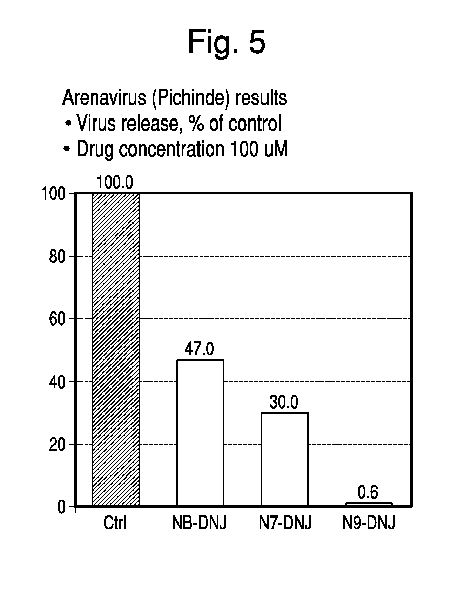

Figure 5 presents data on the inhibition of Pichinde virus release by NB-DNJ; N7-O-DNJ and N9-DNJ.

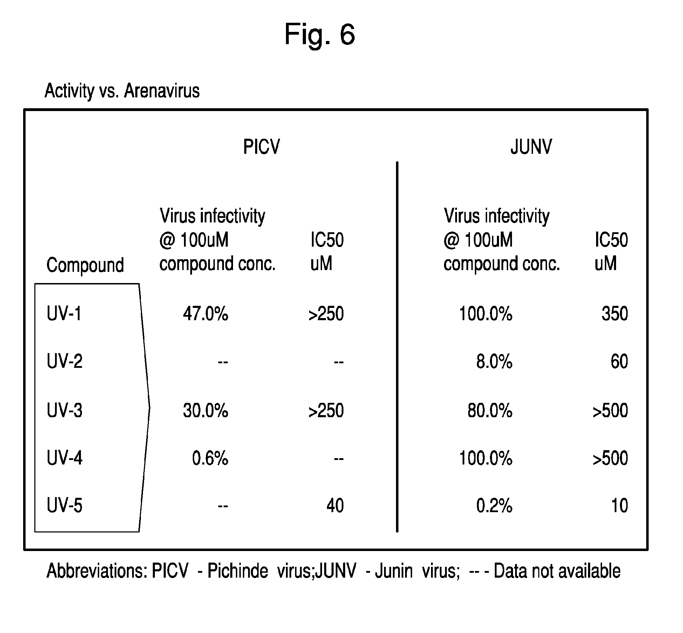

Figure 6 presents activity of selected iminosugars against Pichinde virus (PICV) and Junin virus (JUNV).

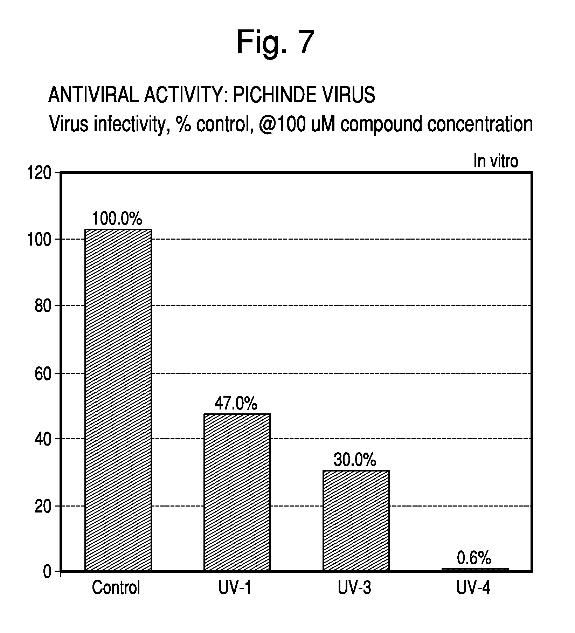

Figure 7 presents antiviral activity of NB-DNJ, N7-O-DNJ and N9-DNJ against PICV.

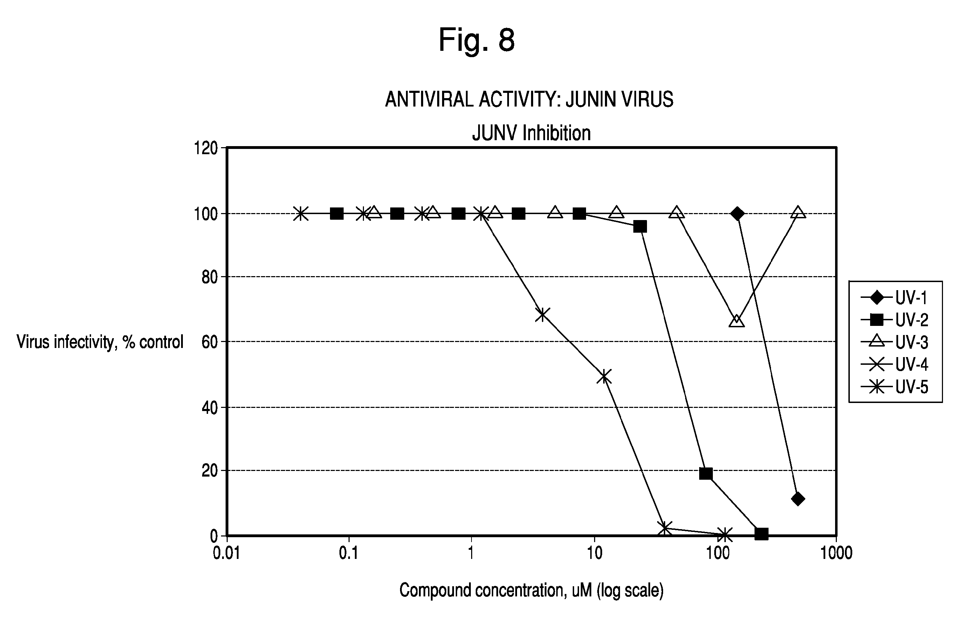

Figure 8 presents antiviral activity of NB-DNJ, NN-DNJ, N7-O-DNJ, N9-DNJ and NAP-DNJ against JUNV.

### DETAILED DESCRIPTION

### Definition of terms

Unless otherwise specified, "a" or "an" means "one or more."

As used herein, the term "viral infection" describes a diseased state, in which a virus invades a healthy cell, uses the cell's reproductive machinery to multiply or replicate and ultimately lyse the cell resulting in cell death, release of viral particles and the infection of other cells by the newly produced progeny viruses. Latent infection by certain viruses is also a possible result of viral infection.

As used herein, the term "treating or preventing viral infection" means to inhibit the replication of the particular virus, to inhibit viral transmission, or to prevent the virus from establishing itself in its host, and to ameliorate or alleviate the symptoms of the disease caused by the viral infection. The treatment is considered therapeutic if there is a reduction in viral load, decrease in mortality and/or morbidity.

IC50 or IC90 (inhibitory concentration 50 or 90) is a concentration of a therapeutic agent, such as an iminosugar, used to achieve 50% or 90% reduction of viral load, respectively.

### Related Applications

The present application refers to by reference in its entirety U.S. provisional application no. 61/202,391 filed February 24, 2009.

### Disclosure

The present inventors discovered that certain iminosugars, such as deoxynojirimycin derivatives, can be effective against viruses belonging to the Arenaviridae family.

In particular, the deoxynojirimycin derivatives can be useful for treating or preventing a disease or condition caused by or associated with a virus belonging to the Arenaviridae family.

Viruses belonging to the Arenaviridae family include arenaviruses belonging to the genus Arenavirus. Arenaviruses may cause a number of viral hemorrhagic fevers. The Arenavirus genus includes Ippy virus; Lassa virus; Lymphocytic choriomeningitis virus; Mobala virus; Mopeia virus; Amapari virus; Flexal virus; Guanarito virus; Junin virus; Latino virus; Machupo virus; Oliveros virus; Paraná virus; Pichinde virus; Pirital virus; Sabiá virus; Tacaribe virus; Tamiami virus; Whitewater Arroyo virus; and Chapare virus. Arenaviruses can be often transmitted by contact with rodents which can serve as the virus reservoir. Arenavirus infections can be endemic worldwide, and cause more than 1 million cases each year, with thousands of deaths. Pichinde virus, a member of the Arenavirus genus, which is not as dangerous to humans as other arenaviruses, is frequently used as a model in order to test activity of chemical compounds against this genus.

The diseases caused by or associated with arenaviruses include Lymphocytic choriomeningitis caused by Lymphocytic choriomeningitis virus; Lassa fever caused by Lassa virus; Argentine hemorrhagic fever caused by Junin virus; Bolivian hemorrhagic fever caused by Machupo virus; Venezuelan hemorrhagic fever caused by Guaranito virus; Brazilian hemorrhagic fever caused by Sabia virus; Tacaribe fever associated with Tacaribe virus; Influenza-like illness associated with Flexal virus; Hemorragic fever associated with Whitewate Arroyo virus.

In many embodiments, the iminosugar may be N-substituted deoxynojirimycin. In some embodiments, such N-substituted deoxynojirimycin may be a compound of the following formula: 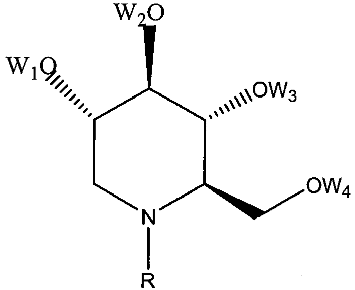 where W₁₋₄ are independently selected from hydrogen, substituted or unsubstituted alkyl groups, substituted or unsubstituted haloalkyl groups, substituted or unsubstituted alkanoyl groups, substituted or unsubstituted aroyl groups, or substituted or unsubstituted haloalkanoyl groups.

In some embodiments, R may be selected from substituted or unsubstituted alkyl groups, substituted or unsubstituted cycloalkyl groups, substituted or unsubstituted aryl groups, or substituted or unsubstituted oxaalkyl groups.

In some embodiments, R may be substituted or unsubstituted alkyl groups and/or substituted or unsubstituted oxaalkyl groups comprise from 1 to 16 carbon atoms, from 4 to 12 carbon atoms or from 8 to 10 carbon atoms. The term "oxaalkyl" refers to an alkyl derivative, which may contain from 1 to 5 or from 1 to 3 or from 1 to 2 oxygen atoms. The term "oxaalkyl" includes hydroxyterminated and methoxyterminated alkyl derivatives.

In some embodiments, R may be selected from, but is not limited to -(CH₂)₆OCH₃, -(CH₂)₆OCH₂CH₃, -(CH₂)₆O(CH₂)₂CH₃, -(CH₂)₆O(CH₂)₃CH₃, -(CH₂)₂O(CH₂)₅CH₃, -(CH₂)₂O(CH₂)₆CH₃,;-(CH₂)₂O(CH₂)₇CH₃; -(CH₂)₉-OH; -(CH₂)₉OCH₃.

In some embodiments, R may be branched or unbranched, substituted or unsubstituted alkyl group. In certain embodiments, the alkyl group may be a long chain alkyl group, which may be C6-C20 alkyl group; C8-C16 alkyl group; or C8-C10 alkyl group. In some embodiments, R may be a long chain oxaalkyl group, i.e. a long chain alkyl group, which may contain from 1 to 5 or from 1 to 3 or from 1 to 2 oxygen atoms.

In some embodiments, R may have the following formula 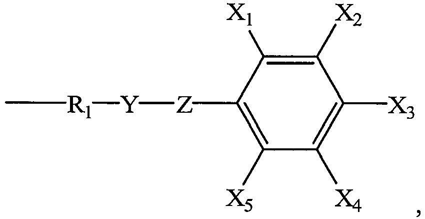 where R₁ is a substituted or unsubstituted alkyl group;
X₁₋₅ are independently selected from H, NO₂, N₃, or NH₂;
Y is absent or is a substituted or unsubstituted C₁-alkyl group, other than carbonyl; and
Z is selected from a bond or NH; provided that when Z is a bond, Y is absent, and provided that when Z is NH, Y is a substituted or unsubstituted C₁-alkyl group, other than carbonyl.

In some embodiments, Z is NH and R₁-Y is a substituted or unsubstituted alkyl group, such as C2-C20 alkyl group or C4-C12 alkyl group or C4-C10 alkyl group.

In some embodiments, X₁ is NO₂ and X₃ is N₃. In some embodiments, each of X₂, X₄ and X₅ is hydrogen.

In some embodiments, the iminosugar may be a DNJ derivative disclosed in U.S. Patent application publication no. 2007/0275998, which is incorporated herein by reference.

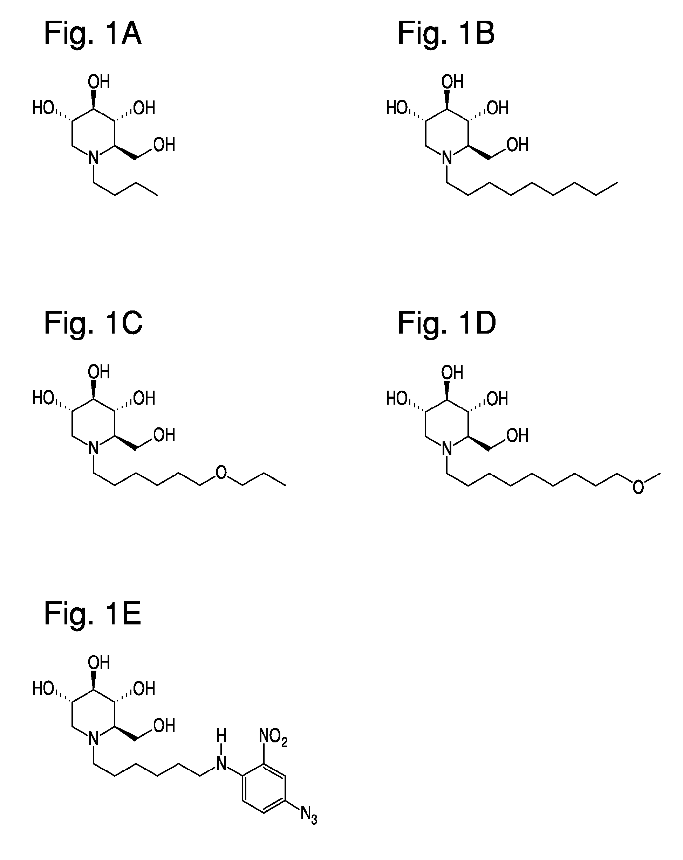

In some embodiments, the deoxynojirimycin derivative may be one of the compounds presented in Figure 1.

Methods of synthesizing deoxynojirimycin derivatives are disclosed, for example, in U.S.

Patent Nos. 5,622,972, 5,200,523, 5,043,273, 4,994,572, 4,246,345, 4,266,025, 4,405,714, and 4,806,650 and U.S. Patent application publication no. 2007/0275998, which are all incorporated herein by reference.

In some embodiments, the iminosugar may be in a form of a salt derived from an inorganic or organic acid. Pharmaceutically acceptable salts and methods for preparing salt forms are disclosed, for example, in Berge et al. (J. Pharm. Sci. 66:1-18, 1977). Examples of appropriate salts include but are not limited to the following salts: acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glucoheptanoate, glycerophosphate, hemisulfate, heptanoate, hexanoate, fumarate, hydrochloride, hydrobromide, hydroiodide, 2-hydroxyethanesulfonate, lactate, maleate, methanesulfonate, nicotinate, 2-naphthalenesulfonate, oxalate, palmoate, pectinate, persulfate, 3-phenylpropionate, picrate, pivalate, propionate, succinate, tartrate, thiocyanate, tosylate, mesylate, and undecanoate.

In some embodiments, the iminosugar may also used in a form of a prodrug. Prodrugs of DNJ derivatives, such as the 6-phosphorylated DNJ derivatives, are disclosed in U.S. Patents nos. 5,043,273 and 5,103,008.

In some embodiments, the iminosugar may be used as a part of a composition, which further comprises a pharmaceutically acceptable carrier and/ or a component useful for delivering the composition to an animal. Numerous pharmaceutically acceptable carriers useful for delivering the compositions to a human and components useful for delivering the composition to other animals such as cattle are known in the art. Addition of such carriers and components to the composition of the invention is well within the level of ordinary skill in the art.

In some embodiments, the pharmaceutical composition may consist essentially of *N-*substituted deoxynojirimycin, which may mean that the *N*-substituted deoxynojirimycin is the only active ingredient in the composition.

Yet in some embodiments, *N*-substituted deoxynojirimycin may be administered with one or more additional antiviral compounds.

In some embodiments, the iminosugar may be used in a liposome composition, such as those disclosed in US publication 2008/0138351; US application No. 12/410,750 filed March 25, 2009 and US provisional application No. 61/202,699 filed March 27, 2009.

The iminosugar, such as a DNJ derivative, may be administered to a cell or an animal affected by a virus. The iminosugar may inhibit morphogenesis of the virus, or it may treat the individual. The treatment may reduce, abate, or diminish the virus infection in the animal.

Animals that may be infected with a virus that belongs to the Arenaviridae family, include vertebrates, such as birds and mammals including primates, humans, rodents and bats.

The amount of iminosugar administered to an animal or to an animal cell to the methods of the invention may be an amount effective to inhibit the morphogenesis of a virus belonging to the Arenaviridae family from the cell. The term "inhibit" as used herein may refer to the detectable reduction and/or elimination of a biological activity exhibited in the absence of the iminosugar. The term "effective amount" may refer to that amount of the iminosugar necessary to achieve the indicated effect. The term "treatment" as used herein may refer to reducing or alleviating symptoms in a subject, preventing symptoms from worsening or progressing, inhibition or elimination of the causative agent, or prevention of the infection or disorder related to the virus belonging to the Arenaviridae family in a subject who is free therefrom.

Thus, for example, treatment of the disease caused by or associated with a virus may include destruction of the infecting agent, inhibition of or interference with its growth or maturation, and neutralization of its pathological effects. The amount of the iminosugar which may be administered to the cell or animal is preferably an amount that does not induce any toxic effects which outweigh the advantages which accompany its administration.

Actual dosage levels of active ingredients in the pharmaceutical compositions may vary so as to administer an amount of the active compound(s) that is effective to achieve the desired therapeutic response for a particular patient.

The selected dose level may depend on the activity of the iminosugar, the route of administration, the severity of the condition being treated, and the condition and prior medical history of the patient being treated. However, it is within the skill of the art to start doses of the compound(s) at levels lower than required to achieve the desired therapeutic effect and to gradually increase the dosage until the desired effect is achieved. If desired, the effective daily dose may be divided into multiple doses for purposes of administration, for example, two to four doses per day. It will be understood, however, that the specific dose level for any particular patient may depend on a variety of factors, including the body weight, general health, diet, time and route of administration and combination with other therapeutic agents and the severity of the condition or disease being treated. The adult human daily dosage may range from between about one microgram to about one gram, or from between about 10 mg and 100 mg, of the iminosugar per 10 kilogram body weight. Of course, the amount of the iminosugar which should be administered to a cell or animal may depend upon numerous factors well understood by one of skill in the art, such as the molecular weight of the iminosugar and the route of administration.

Pharmaceutical compositions that are useful in the methods of the invention may be administered systemically in oral solid formulations, ophthalmic, suppository, aerosol, topical or other similar formulations. For example, it may be in the physical form of a powder, tablet, capsule, lozenge, gel, solution, suspension, syrup, or the like. In addition to the iminosugar, such pharmaceutical compositions may contain pharmaceutically-acceptable carriers and other ingredients known to enhance and facilitate drug administration. Other possible formulations, such as nanoparticles, liposomes, resealed erythrocytes, and immunologically based systems may also be used to administer the iminosugar. Such pharmaceutical compositions may be administered by a number of routes. The term "parenteral" used herein includes subcutaneous, intravenous, intraarterial, intrathecal, and injection and infusion techniques, without limitation. By way of example, the pharmaceutical compositions may be administered orally, topically, parenterally, systemically, or by a pulmonary route.

These compositions may be administered a in a single dose or in multiple doses which are administered at different times. Because the inhibitory effect of the composition upon a virus belonging to the Arenaviridae family may persist, the dosing regimen may be adjusted such that virus propagation is retarded while the host cell is minimally effected. By way of example, an animal may be administered a dose of the composition of the invention once per week, whereby virus propagation is retarded for the entire week, while host cell functions are inhibited only for a short period once per week.

Embodiments described herein are further illustrated by, though in no way limited to, the following working examples.

### Working Examples

### 1. Synthesis of N-Nonyl DNJ

**Table 1. Materials for NN-DNJ synthesis**

| Name | Amount |
|---|---|
| DNJ | 500 mg |
| Nonanal | 530 mg |
| Ethanol | 100 mL |
| AcOH | 0.5 mL |
| Pd/C | 500 mg |

Procedure: A 50-mL, one-necked, round-bottom flask equipped with a magnetic stirrer was charged with DNJ (500 mg), ethanol (100 mL), nonanal (530 mg), and acetic acid (0.5 mL) at room temperature. The reaction mixture was heated to 40-45 °C and stirred for 30-40 minutes under nitrogen. The reaction mixture was cooled to ambient temperature and Pd/C was added. The reaction flask was evacuated and replaced by hydrogen gas in a balloon. This process was repeated three times. Finally, the reaction mixture was stirred at ambient temperature overnight. The progress of reaction was monitored by TLC (Note 1). The reaction mixture was filtered through a pad of Celite and washed with ethanol. The filtrate was concentrated *in vacuo* to get the crude product. The crude product was purified by column chromatography (230-400 mesh silica gel). A solvent gradient of methanol in dichloromethane (10-25%) was used to elute the product from the column. All fractions containing the desired product were combined, and concentrated *in vacuo* to give the pure product (420mg). Completion of the reaction was monitored by thin layer chromatography (TLC) using a thin layer silica gel plate; eluent; methanol: dichloromethane = 1:2

### 2. Synthesis of N-7-Oxadecyl DNJ

### 2a. Synthesis of 6-propyloxy-1-hexanol

**Table 2. Materials for synthesis of 6-propyloxy-1-hexanol**

| Name | Amount |
|---|---|
| 1,6-hexanediol | 6.00 g |
| 1-Iodopropane | 8.63 g |
| Potassium tert-butoxide | 5.413 mg |
| THF | 140 mL |

Procedure: a 500-mL, one-necked, round-bottom flask equipped with a magnetic stirrer was charged with 1,6-hexanediol (6.00 g), potassium tert-butoxide (5.413 g) at room temperature. The reaction mixture was stirred for one hour, and then 1-iodopropane (8.63 g) was added. The reaction mixture was heated to 70-80 °C and stirred overnight. The progress of reaction was monitored by TLC (Note 1). After completion of the reaction, water was added to the reaction mixture, and extracted with ethyl acetate (2 x 100 mL). The combined organic layers were concentrated *in vacuo* to get the crude product. The crude product was dissolved in dichloromethane and washed with water, and then brine, dried over sodium sulfate. The organic layer was concentrated *in vacuo* to get the crude product. The crude product was purified by column chromatography using 230-400 mesh silica gel. A solvent gradient of ethyl acetate in hexanes (10-45%) was used to elute the product from the column. All fractions containing the desired pure product were combined and concentrated *in vacuo* to give pure 6-propyloxy-1-hexanol (lot D-1029-048, 1.9 g, 25%) Completion of the reaction was monitored by thin layer chromatography (TLC); (eluent: 60% ethyl acetate in hexanes).

### 2b. Preparation of 6-propyloxy-1-hexanal

**Table 3. Materials for preparation of 6-propyloxy-1-hexanal**

| Name | Amount |
|---|---|
| 6-Propyloxy-1-hexanol | 1.00 g |
| PDC | 4.70 g |
| Celite | 1.00 g |
| NaOAc | 100 mg |
| CH₂Cl₂ | 10 mL |

Procedure: a 50-mL, one-necked, round-bottom flask equipped with a magnetic stirrer was charged with 6-propyloxy-1-hexanol (1.0 g), PDC (4.7 g), dichloromethane (10 mL), Celite (1.0 g), and sodium acetate (100 mg). The reaction mixture was stirred at room temperature under nitrogen for 5 minutes. PDC (4.70 g) was added to the reaction mixture, and stirred overnight. The progress of reaction was monitored by TLC (Note 1). After completion of the reaction, the reaction mixture was directly loaded on the column (230-400 mesh silica gel). A solvent gradient of dichloromethane in ethyl acetate (10-20%) was used to elute the product from the column. All fractions containing the desired pure product were combined and concentrated *in vacuo* to give pure 6-propyloxy-1-hexanal (lot D-1029-050, 710 mg, 71 %). Completion of the reaction was monitored by thin layer chromatography (TLC); (eluent: 60% ethyl acetate in hexanes).

### 2c Synthesis of N-7-Oxadecyl-DNJ

**Table 4. Materials for Synthesis of N-7-Oxadecyl-DNJ**

| Name | Amount |
|---|---|
| DNJ | 500 mg |
| 6-Propyloxy-1-hexanal | 585 mg |
| Pd/C | 125 mg |
| Ethanol | 15 mL |
| Acetic acid | mL |

Procedure: a 50-mL, one-necked, round-bottom flask equipped with a magnetic stirrer was charged with DNJ (500 mg), ethanol (15 mL), 6-propyloxy-1-hexanal (585 mg), and acetic acid (0.1 mL) t room temperature. The reaction mixture was heated to 40-45 °C and stirred for 30-40 minutes under nitrogen. The reaction mixture was cooled to ambient temperature and Pd/C was added. The reaction flask was evacuated and replaced by hydrogen gas in a balloon. This process was repeated three times. Finally, the reaction mixture was stirred at ambient temperature overnight. The progress of reaction was monitored by TLC (Note 1). The reaction mixture was filtered through a pad of Celite and washed with ethanol. The filtrate was concentrated *in vacuo* to get the crude product. The crude product was purified by column chromatography (230-400 mesh silica gel). A solvent gradient of methanol in dichloromethane (10-40%) was used to elute the product from the column. All fractions containing the desired product were combined, and concentrated *in vacuo* to give the pure product. (Lot: D-1029-052 (840 mg). Completion of the reaction was monitored by thin layer chromatography (TLC); (eluent: 50% methanol in dichloromethane).

### 3. Synthesis of N-(9-methoxy)-nonyl DNJ

### 3 a Preparation of 9-methoxy-1-nonanol

**Table 5. Materials for preparation of 9-methoxy-1-nonanol**

| Name | Amount |
|---|---|
| 1,9-nonanediol | 10.0 g |
| Dimethyl sulfate | 41.39 g |
| Sodium hydroxide | 5.0g |
| DMSO | 100 mL |

Procedure: a 500-mL, one-necked, round-bottom flask equipped with a magnetic stirrer and stir bar was charged with 1,9-nonanediol (10.00 g, 62.3 mmol) in dimethyl sulfoxide (100 mL) and H₂O (100 mL). To this was added slowly a solution of sodium hydroxide (5.0 g, 125.0 mmol) in H₂O (10 mL) at room temperature. During addition of sodium hydroxide the reaction mixture generated heat and the temperature rose to ~40 °C. The mixture was stirred for one hour, and then dimethyl sulfate (16.52 g, 131 mmol) was added in four portions while maintaining the temperature of the reaction mixture at ~ 40°C. The reaction mixture was stirred at room temperature overnight. Progress of the reaction was monitored by TLC (Note 1). TLC monitoring indicated that the reaction was 25 % conversion. At this stage additional dimethyl sulfate (24.78g, 196.44 mmol) was added and the resulting mixture was stirred at room temperature for an additional 24 h. After completion of the reaction, sodium hydroxide (10% solution in water) was added to the reaction mixture to adjust the pH of the solution to 11-13. The mixture was stirred at room temperature for 2 h and extracted with dichloromethane (3 x 100 mL). The combined organic layers were washed with H₂O (200 mL), brine (150 mL), dried over anhydrous sodium sulfate (20 g), filtered and concentrated *in vacuo* to obtain a crude product (14 g). The crude product was purified by column chromatography using 250-400 mesh silica gel. A solvent gradient of ethyl acetate in hexanes (10-50%) was used to elute the product from the column. All fractions containing the desired pure product were combined and concentrated *in vacuo* to give pure 9-methoxy-1-nonanol (lot D-1027-155, 2.38 g, 21.9 %). Completion of the reaction was monitored by thin layer chromatography (TLC) using a thin layer silica gel plate; eluent: 60% ethyl acetate in hexanes.

### 3b Preparation of 9-methoxy-1-nonanal

**Table 6. Materials for preparation of 9-methoxy-1-nonanal**

| Name | Amount |
|---|---|
| 9-methoxy-1-nonanol | 1.0 g |
| PDC | 4.7 g |
| Molecular sieves, 3A | 1.0 g |
| NaOAc | 0.1g |
| CH₂Cl₂ | 10 mL |

Procedure: a 50-mL, one-necked, round-bottom flask equipped with a magnetic stirrer and stir bar was charged with 9-methoxy-nonanol (1.0 g, 5.9 mmol), dichloromethane (10 mL), molecular sieves (1.0 g, 3A), sodium acetate (0.1 g) at room temperature. The reaction mixture was stirred at room temperature under nitrogen for 5 minutes. The reaction mixture was charged with pyridinium dichromate (4.7 g, 12.5 mmol) and stirred overnight. The progress of reaction was monitored by TLC (Note 1). After completion of the reaction, the reaction mixture was filtered through a bed of silica gel (~15 g). The filtrate was evaporated in *vacuo* to obtain a crude compound. This was purified by column chromatography using silica gel column (250-400 mesh, 40 g). A solvent gradient of ethyl acetate in hexane (10-50%) was used to elute the product from the column. All fractions containing the desired pure product were combined and concentrated *in vacuo* to give pure 9-methoxy-nonanal (lot D-1027-156, 553 mg, 54.4%). Completion of the reaction was monitored by thin layer chromatography (TLC) using a thin layer silica gel plate; eluent: 60% ethyl acetate in hexanes.

### 3c Synthesis of N-(9-methoxy)-nonyl DNJ

**Table 7. Materials for synthesis of N-(9-methoxy)-nonyl DNJ**

| Name | Amount |
|---|---|
| DNJ | 300 mg |
| 9-methoxy-1-nonanal | 476 mg |
| Pd/C | 200 mg |
| Ethanol | 20 mL |

Procedure: a 50-mL, two-necked, round-bottom flask equipped with magnetic stirrer and a stir bar was charged with DNJ (300 mg, 1.84 mmol), ethanol (20 mL), 9-methoxy-1-nonanal (476 mg, 2.76 mmol) at room temperature. The reaction mixture was stirred for 5-10 minutes under nitrogen and Pd/C was added at room temperature. The reaction mixture was evacuated and was replaced by hydrogen gas using a balloon. This process was repeated three times and then reaction mixture was stirred under atmospheric hydrogen at room temperature. The progress of reaction was monitored by TLC (Note 1). The reaction mixture was filtered through a bed of Celite and was washed with ethanol (20 mL). The filtrate was concentrated *in vacuo* to get a crude product. The crude product was purified by column chromatography using 250-400 mesh silica gel (20 g). A solvent gradient of methanol in ethyl acetate (5-25%) was used to elute the product from the column. All fractions containing the desired pure product were combined, and concentrated *in vacuo* to give an off white solid. The solid was triturated in ethyl acetate (20 mL), filtered and dried in high vacuum to give a white solid [lot: D-1027-158 (165.3 mg, 28.1 %). Completion of the reaction was monitored by thin layer chromatography (TLC) using a thin layer silica gel plate; eluent: 50% methanol in dichloromethane.

### 4. Effect of iminosugars against Pichinde Virus

Figure 5 presents data on the inhibition of Pichinde virus release by the following UV iminosugar compounds: NB-DNJ (UV-1); NN-DNJ (UV-2); N7-O-DNJ (UV-3); N9-DNJ (UV-4); NAP-DNJ (UV-5). Control Vero cell cultures and Vero cell cultures treated with 100µM compounds were infected with virus and cultured for 7 days at 37°C in a 5% CO2 incubator. Inhibition of production of infectious virus particles from virus infected cell cultures treated with compounds were determined by plaque assay.

The virus plaque assay was performed in Vero cells plated in 6-well plates at 5x10⁵ cells per well in 1x modified Eagle medium (Gibco), supplemented with 2% fetal bovine serum, 2 mM L-glutamine, 100 U/ml penicillin, 100 µg/ml streptomycin. The virus to be titered from collected supernatants from infected cell cultures treated with the compounds were diluted in cell culture medium and inoculated in 100 µl volumes onto cells and allowed to adsorb for 1 hr at 37°C. The cells were overlaid with 0.6% agarose in 1x modified Eagle medium (Gibco), supplemented with 2 mM L-glutamine, 100 U/ml penicillin, 100 µg/ml streptomycin.

Plaques, of dead cells representing individual infectious virus particles that has infected and killed cells, were allowed to develop at 37°C in a 5% CO2 incubator and visualized by live-staining the cell monolayer with neutral red. The experiment demonstrates that release of infectious Pichinde virus is significantly reduced after treatment with UV iminosugar compounds.

### 5. Effects of iminosugars against Pichinde and Junin viruses

Figure 6 presents data on the inhibition of Pichinde virus and Junin virus releases by the following UV iminosugar compounds: NB-DNJ (UV-1); NN-DNJ (UV-2); N7-O-DNJ (UV-3); N9-DNJ (UV-4); NAP-DNJ (UV-5).

Compounds. Base stocks of the following compounds were prepared in dimethylsulfoxide (DMSO) to a final maximal DMSO concentration of 0.5%: UV-1, UV-2, UV-3, UV-4, and UV-5. All compounds were diluted from the base stocks to their experimental concentrations.

Viruses. The compounds were screened for inhibition against Pichinde Virus (Arenavirus) CoAn 3739 strain, and the Junin (Arenavirus) Candid #1 strain. Viral stocks were made by propagation in Vero cells using modified Eagle medium (MEM, Sigma), supplemented with 2% fetal bovine serum, 2 mM L-glutamine, 100 U/ml penicillin, 100 µg/ml streptomycin and titered using the standard plaque assay (method presented below). Viral stocks were stored at -80°C until used.

Virus Yield Reduction Assay. The virus yield assay were performed by standard plaque assay on supernatant samples generated from virus-infected cells incubated with different concentrations of iminosugars. 24-well cell culture plates were seeded with cells in 1mL MEM with 10% fetal bovine serum Vero cells (ATCC, Mannassas, VA; ATCC number CCL-81) in MEM with Earl's salts (Sigma, St Louis, MO) supplemented with 2 mM L-glutamine, 100 U/mL penicillin/streptomycin, and 2% heat-inactivated fetal bovine serum and incubated at 37°C for 24 hours or until ~80% confluency. Medium were replaced with medium supplemented with 2% fetal bovine serum and the compound concentrations to be used started at 500 µM, 250 µM or 125 µM and tested in triplicate using 8 dilutions.

Compounds are added to appropriate wells and incubated for 1hr at 37°C, 5% CO₂. After 1hr incubation virus is added to each well. Four days are required for the PICV and five days for JUNV virus infection. Upon completion of infection, supernatant were collected for titering. To titer PICV and JUNV, 12-well plates with 80% confluent Vero cells in growth medium were used. Viral supernatant were diluted from 10⁻³ to 10⁻⁸ and added (100uL) to the cells and incubated at 37°C for 1 hour with shaking every 5-10 minutes. Viral infection medium (100uL) were aspirated and replace with 1mL pre-warmed 2% low-melt agarose mixed 1:1 with 2X MEM (5% fetal calf serum) and incubated at 37°C, 5% CO₂ for 6 days followed by plaque visualization by neutral red staining. IC 50 was determined as concentration of compound resulting in 50% virus inhibition.

Figure 7 compares inhibition of Pichinde virus for control, UV-1, UV-3 and UV-4. Compounds. Base stocks of the following compounds were prepared in dimethylsulfoxide (DMSO) to a final maximal DMSO concentration of 0.5%: UV-1, UV-2, UV-3, UV-4, UV-5. All compounds were diluted from the base stocks to their experimental concentrations. Virus. The compounds were screened for inhibition against the Pichinde virus CoAn 3739 strain.

Results: The virus yield assay were performed as described above. PICV CoAn 3739 strain virus inhibition was found for compounds NB-DNJ, N7-O-DNJ, and N9-DNJ. PICV in vitro inhibition resulted in over 50% inhibition by NB-DNJ, 70% inhibition by N7-O-DNJ and over 99% inhibition by N9-DNJ at a 100 µM iminosugar concentration.

Figure 8 shows inhibition plots for Junin virus by UV-1, UV-2, UV-3, UV-4 and UV-5 iminosugars as percentage of viral infectivity compared with control versus concentration of iminosugar compound.

Compounds. Base stocks of the following compounds were prepared in dimethylsulfoxide (DMSO) to a final maximal DMSO concentration of 0.5%: UV-1, UV-2, UV-3, UV-4, UV-5. All compounds were diluted from the base stocks to their experimental concentrations.

Virus. The compounds were screened against the Junin virus Candid #1 strain.

Results: The virus yield assay were performed as described above. Junin virus inhibition was found for compounds NB-DNJ with an EC50 of 350 µM. NN-DNJ with an EC50 of 60 µM, and NAP-DNJ showed protection with and EC50 of 10 µM. Compounds N7-O-DNJ and N9-DNJ had EC50s over 500 µM.

Although the foregoing refers to particular preferred embodiments, it will be understood that the present invention is not so limited. It will occur to those of ordinary skill in the art that various modifications may be made to the disclosed embodiments and that such modifications are intended to be within the scope of the present invention.

All of the publications, patent applications and patents cited in this specification are incorporated herein by reference in their entirety.

### Aspects of the invention are:

Aspect 1. A method of treating or preventing a disease or condition caused by or associated with a virus belonging to the Arenaviridae family, the method comprising administering to a subject in need thereof an effective amount of a compound of the formula, 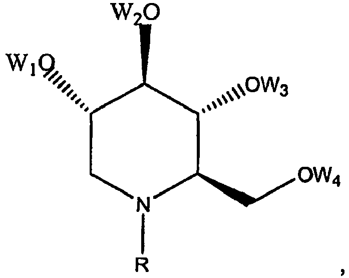 or a pharmaceutically acceptable salt thereof,
   wherein R is either selected from substituted or unsubstituted alkyl groups, substituted or unsubstituted cycloalkyl groups, substituted or unsubstituted aryl groups, or substituted or unsubstituted oxaalkyl groups; or wherein R is 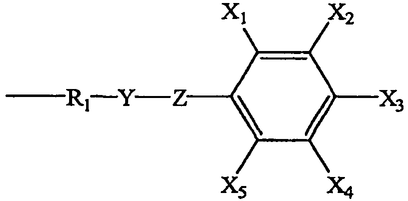 R₁ is a substituted or unsubstituted alkyl group;
   X₁₋₅ are independently selected from H, NO₂, N₃, or NH₂;
   Y is absent or is a substituted or unsubstituted C₁-alkyl group, other than carbonyl; and
   Z is selected from a bond or NH; provided that when Z is a bond, Y is absent, and provided that when Z is NH, Y is a substituted or unsubstituted C₁-alkyl group, other than carbonyl; and
   wherein W₁₋₄ are independently selected from hydrogen, substituted or unsubstituted alkyl groups, substituted or unsubstituted haloalkyl groups, substituted or unsubstituted alkanoyl groups, substituted or unsubstituted aroyl groups, or substituted or unsubstituted haloalkanoyl groups.
Aspect 2. The method of aspect 1, wherein each of W₁, W₂, W₃ and W₄ is hydrogen.
Aspect 3. The method of aspect 1, wherein R is selected from substituted or unsubstituted alkyl groups, substituted or unsubstituted cycloalkyl groups, substituted or unsubstituted aryl groups, or substituted or unsubstituted oxaalkyl groups.
Aspect 4. The method of aspect 1, wherein R is C2-C12 alkyl group.
Aspect 5. The method of aspect 1, wherein R is C3-C6 alkyl group.
Aspect 6. The method of aspect 1, wherein said administering comprises administering B-butyl deoxynojirimycin or a pharmaceutically acceptable salt thereof.
Aspect 7. The method of aspect 1, wherein R is an oxaalkyl group.
Aspect 8. The method of aspect 1, wherein R is C2-C16 oxaalkyl group that contains from 1 to 3 oxygen atoms.
Aspect 9. The method of aspect 1, wherein R is C6-C12 oxaalkyl group that contains from 1 to 2 oxygen atoms.
Aspect 10. The method of aspect 1, wherein said administering comprises administering N-(7-oxadecyl)deoxynojirimycin or a pharmaceutically acceptable salt thereof.
Aspect 11. The method of aspect 1, wherein said administering comprises administering is N-(9-Methoxynonyl)deoxynojirimycin or a pharmaceutically acceptable salt thereof.
Aspect 12. The method of aspect 1, wherein R is 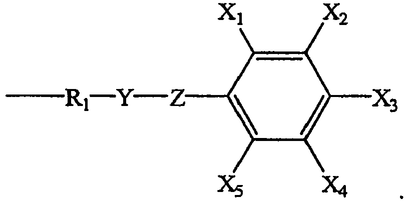
Aspect 13. The method of aspect 12, wherein X₁ is NO₂ and X₃ is N₃.
Aspect 14. The method of aspect 12, wherein each of X₂, X₄ and X₅ is hydrogen.
Aspect 15. The method of aspect 1, wherein said administering comprises administering is *N*-(*N*-{4'-azido-2'-nitrophenyl}-6-aminohexyl)deoxynojirimycin or a pharmaceutically acceptable salt thereof.
Aspect 16. The method of aspect 1, wherein the subject is a mammal.
Aspect 17. The method of aspect 1, wherein the subject is a human being.
Aspect 18. The method of aspect 1, wherein the virus is selected from Ippy virus; Lassa virus; Lymphocytic choriomeningitis virus; Mobala virus; Mopeia virus; Amapari virus; Flexal virus; Guanarito virus; Junin virus; Latino virus; Machupo virus; Oliveros virus; Paraná virus; Pichinde virus; Pirital virus; Sabiá virus; Tacaribe virus; Tamiami virus; Whitewater Arroyo virus; and Chapare virus.
Aspect 19. The method of aspect 1, wherein the virus is Pichinde virus.
Aspect 20. The method of aspect 1, wherein the virus is Junin virus.
Aspect 21. The method of aspect 1, wherein the disease or condition is selected from Lymphocytic choriomeningitis; Lassa fever; Argentine hemorroagic fever; Bolivian hemorrhagic fever; Brazilian hemorrhagic fever; Tacaribe fever; Venezuelan hemorrhagic fever; Influenza-like illness associated with Flexal virus; and Hemorragic fever associated with Whitewate Arroyo virus.
Aspect 22. The method of aspect 1, wherein the disease or conditions is Argentine hemorrhagic fever.
Aspect 23. The method of aspect 1, wherein the disease or conditions is Lassa fever.

## Claims

1. A compound of the formula: 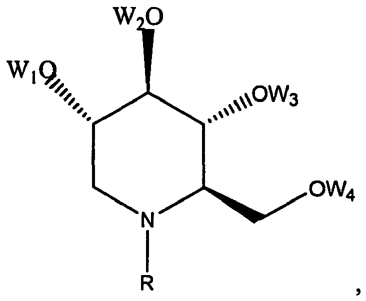 or a pharmaceutically acceptable salt thereof, for use, in an effective amount, in treating or preventing a disease or condition caused by or associated with a virus belonging to the Arenaviridae family in a subject,
wherein R is either selected from substituted or unsubstituted alkyl groups, substituted or unsubstituted cycloalkyl groups, substituted or unsubstituted aryl groups, or substituted or unsubstituted oxaalkyl groups; or wherein R is 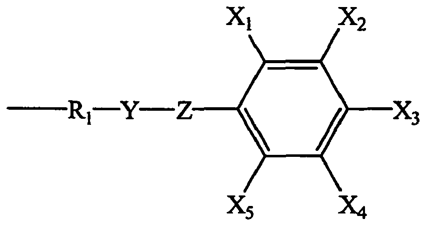 R₁ is a substituted or unsubstituted alkyl group;
X₁₋₅ are independently selected from H, NO₂, N₃, or NH₂;
Y is absent or is a substituted or unsubstituted C₁-alkyl group, other than carbonyl;
and
Z is selected from a bond or NH; provided that when Z is a bond, Y is absent, and provided that when Z is NH, Y is a substituted or unsubstituted C₁-alkyl group, other than carbonyl; and
wherein W₁₋₄ are independently selected from hydrogen, substituted or unsubstituted alkyl groups, substituted or unsubstituted haloalkyl groups, substituted or unsubstituted alkanoyl groups, substituted or unsubstituted aroyl groups, or substituted or unsubstituted haloalkanoyl groups.

2. The compound of claim 1, wherein each of W₁, W₂, W₃ and W₄ is hydrogen.

3. The compound of claim 1, wherein R is selected from substituted or unsubstituted alkyl groups, substituted or unsubstituted cycloalkyl groups, substituted or unsubstituted aryl groups, or substituted or unsubstituted oxaalkyl groups.

4. The compound of claim 1, wherein R is C₂-C₁₂ alkyl group, such as C₃-C₆ alkyl group.

5. The compound of claim 1, wherein said compound is N-butyl deoxynojirimycin or a pharmaceutically acceptable salt thereof.

6. The compound of claim 1, wherein R is an oxaalkyl group, such as C₂-C₁₆ oxaalkyl group that contains from 1 to 3 oxygen atoms or C₆-C₁₂ oxaalkyl group that contains from 1 to 2 oxygen atoms.

7. The compound of claim 1, wherein said compound is N-(7-oxadecyl)deoxynojirimycin or a pharmaceutically acceptable salt thereof.

8. The compound of claim 1, wherein said compound is N-(9-Methoxynonyl)deoxynojirimycin or a pharmaceutically acceptable salt thereof.

9. The compound of claim 1, wherein R is

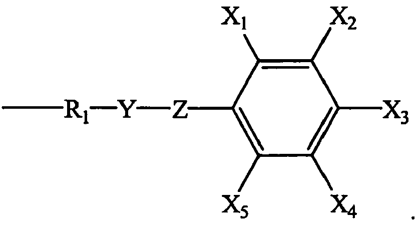

10. The compound of claim 9, wherein X₁ is NO₂ and X₃ is N₃.

11. The compound of claim 9, wherein each of X₂, X₄ and X₅ is hydrogen.

12. The compound of claim 1, wherein said compound is N-(N-{4'-azido-2'-nitrophenyl}-6-aminohexyl)deoxynojirimycin or a pharmaceutically acceptable salt thereof.

13. The compound of claim 1, wherein the subject is a mammal, such as a human being.

14. The compound of claim 1, wherein the virus is selected from Ippy virus; Lassa virus; Lymphocytic choriomeningitis virus; Mobala virus; Mopeia virus; Amapari virus; Flexal virus; Guanarito virus; Junin virus; Latino virus; Machupo virus; Oliveros virus; Paraná virus; Pichinde virus; Pirital virus; Sabiá virus; Tacaribe virus; Tamiami virus; Whitewater Arroyo virus; and Chapare virus.

15. The compound of claim 1, wherein the disease or condition is selected from Lymphocytic choriomeningitis; Lassa fever; Argentine hemorroagic fever; Bolivian hemorrhagic fever; Brazilian hemorrhagic fever; Tacaribe fever; Venezuelan hemorrhagic fever; Influenza-like illness associated with Flexal virus; and Hemorragic fever associated with Whitewate Arroyo virus.

## Patentansprüche

1. Eine Verbindung mit der Formel: 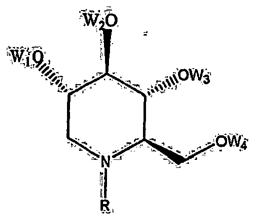 oder ein pharmazeutisch annehmbares Salz davon zur Verwendung, in einer wirksamen Menge, in einem Verfahren zur Behandlung oder Vorbeugung einer Krankheit oder eines Zustands eines Probanden, die/der durch ein zu der Arenaviridae-Familie gehörendes Virus verursacht wird oder damit in Zusammenhang steht,
wobei R aus substituierten oder unsubstituierten Alkylgruppen, substituierten oder unsubstituierten Cycloalkylgruppen, substituierten oder unsubstituierten Arylgruppen oder substituierten oder unsubstituierten Oxaalkylgruppen ausgewählt wird; oder wobei R 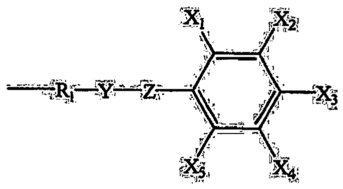 ist, wobei
R₁ eine substituierte oder unsubstituierte Alkylgruppe ist;
X₁₋₅ unabhängig aus H, NO₂, N₃, oder NH₂ ausgewählt werden;
Y nicht vorhanden ist oder eine substituierte oder unsubstituierte C₁-Alkylgruppe ist, die nicht Carbonyl ist, ist;
und
Z aus einer Bindung oder NH ausgewählt wird; vorausgesetzt, dass, wenn Z eine Bindung ist, Y nicht vorhanden ist, und vorausgesetzt, dass, wenn Z NH ist, Y eine substituierte oder unsubstituierte C₁-Alkylgruppe, die nicht Carbonyl ist, ist;
und
wobei W₁₋₄ unabhängig aus Wasserstoff, substituierten oder unsubstituierten Alkylgruppen, substituierten oder unsubstituierten Haloalkylgruppen, substituierten oder unsubstituierten Alkanoylgruppen, substituierten oder unsubstituierten Aroylgruppen oder substituierten oder unsubstituierten Haloalkanoylgruppen ausgewählt werden.

2. Verbindung gemäß Anspruch 1, wobei jedes von W₁, W₂, W₃ und W₄ Wasserstoff ist.

3. Verbindung gemäß Anspruch 1, wobei R aus substituierten oder unsubstituierten Alkylgruppen, substituierten oder unsubstituierten Cycloalkylgruppen, substituierten oder unsubstituierten Arylgruppen oder substituierten oder unsubstituierten Oxaalkylgruppen ausgewählt wird.

4. Verbindung gemäß Anspruch 1, wobei R eine C₂-C₁₂-Alkylgruppe, wie beispielsweise eine C₃-C₆-Alkylgruppe, ist.

5. Verbindung gemäß Anspruch 1, wobei die Verbindung N-Butyldeoxynojirimycin oder ein pharmazeutisch annehmbares Salz davon ist.

6. Verbindung gemäß Anspruch 1, wobei R eine Oxaalkylgruppe ist, wie beispielsweise eine C₂-C₁₆-Oxaalkylgruppe, welche 1 bis 3 Sauerstoffatome enthält, oder eine C₆-C₁₂-Oxaalkylgruppe, welche 1 bis 2 Sauerstoffatome enthält.

7. Verbindung gemäß Anspruch 1, wobei die betreffende Verbindung N-(7-Oxadecyl)deoxynojirimycin oder ein pharmazeutisch annehmbares Salz davon ist.

8. Verbindung gemäß Anspruch 1, wobei die Verbindung N-(9-Methoxynonyl)deoxynojirimycin oder ein pharmazeutisch annehmbares Salz davon ist.

9. Verbindung gemäß Anspruch 1, wobei R 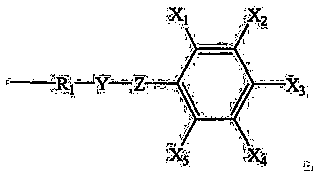 ist.

10. Verbindung gemäß Anspruch 9, wobei X₁ NO₂ und X₃ N₃ ist.

11. Verbindung gemäß Anspruch 9, wobei jedes von X₂, X₄ und X₅ Wasserstoff ist.

12. Verbindung gemäß Anspruch 1, wobei die betreffende Verbindung N-(N-{4'-Azido-2'-Nitrophenyl}-6-Aminohexyl)deoxynojirimycin oder ein pharmazeutisch annehmbares Salz davon ist.

13. Verbindung gemäß Anspruch 1, wobei der Proband ein Säugetier, wie beispielsweise ein Mensch, ist.

14. Verbindung gemäß Anspruch 1, wobei das Virus aus folgenden Viren ausgewählt wird: Ippy-Virus, Lassa-Virus, Lymphozytäre Choriomeningitis-Virus, Mobala-Virus, Mopeia-Virus, Amapari-Virus, Flexal-Virus, Guanarito-Virus, Junin-Virus, Latino-Virus, Machupo-Virus, Oliveros-Virus, Paraná-Virus, Pichinde-Virus, Pirital-Virus, Sabiá-Virus, Tacaribe-Virus, Tamiami-Virus, Whitewater Arroyo-Virus und dem Chapare-Virus.

15. Verbindung gemäß Anspruch 1, wobei die Krankheit oder der Zustand ausgewählt wird aus Lymphozytärer Choriomeningitis, Lassafieber, Argentinischem hämorrhagischem Fieber, Bolivianischem hämorrhagischem Fieber, Brasilianischem hämorrhagischem Fieber, Tacaribefieber, Venezuelanischem hämorrhagischem Fieber, grippeähnlicher Krankheit in Zusammenhang mit einem Flexal-Virus und hämorrhagischem Fieber in Zusammenhang mit einem Whitewater Arroyo-Virus.

## Revendications

1. Un composé de formule : 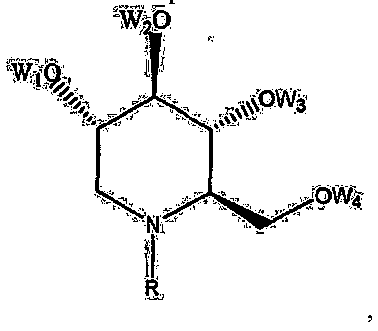 ou un sel pharmaceutiquement acceptable de celui-ci ;
pour l'utilisation, en une quantité efficace, dans le traitement ou la prévention d'une maladie ou d'une condition provoquée par ou associée à un virus appartenant à la famille des Arenaviridae chez un sujet,
dans lequel R est choisi parmi des groupes alkyle substitués ou non substitués, des groupes cycloalkyle substitués ou non substitués, des groupes aryle substitués ou non substitués, ou des groupes oxaalkyle substitués ou non substitués ; ou dans lequel R est 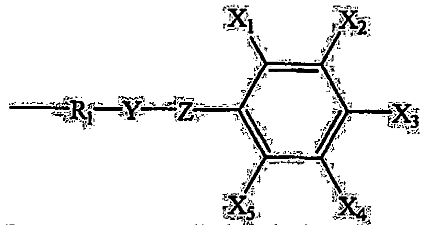 R₁ est un groupe alkyle substitué ou non substitué ;
X1 à X5 sont indépendamment choisis parmi H₃ NO₂, N₃, ou NH₂;
Y est absent ou est un groupe alkyle en C1 substitué ou non substitué, autre que carbonyle ; et
Z est choisi parmi une liaison ou NH ; à condition que lorsque Z est une liaison, Y est absent, et à condition que lorsque Z est NH, Y est un groupe alkyle en C₁ substitué ou non substitué, autre que carbonyle ;
et
dans lequel W_{1 à 4} sont choisis indépendamment entre l'hydrogène, des groupes alkyle substitués ou non substitués, des groupes haloalkyle substitués ou non substitués, des groupes alcanoyle substitués ou non substitués, des groupes aroyle substitués ou non substitués, ou des groupes haloalcanoyle substitués ou non substitués.

2. Le composé selon la revendication 1, dans lequel W₁, W₂, W₃ et W₄ sont chacun un hydrogène.

3. Le composé selon la revendication 1, dans lequel R est choisi parmi des groupes alkyle substitués ou non substitués, des groupes cycloalkyle substitués ou non substitués, des groupes aryle substitués ou non substitués, ou des groupes oxaalkyle substitués ou non substitués.

4. Le composé selon la revendication 1, dans lequel R est un groupe alkyle en C₂ à C₁₂, tel qu'un groupe alkyle en C₃ à C₆.

5. Le composé selon la revendication 1, dans lequel ledit composé est de la N-butyl déoxynojirimycine ou un de ses sels pharmaceutiquement acceptable.

6. Le composé selon la revendication 1, dans lequel R est un groupe oxaalkyle tel qu'un groupe oxaalkyle en C₂ à C₁₆ qui comporte 1 à 3 atomes d'oxygène ou un groupe oxaalkyle en C₆ à C₁₂ qui comporte 1 à 2 atomes d'oxygène.

7. Le composé selon la revendication 1, dans lequel ledit composé est de la N-(7-oxadecyl) déoxynojirimycine ou un de ses sels pharmaceutiquement acceptable.

8. Le composé selon la revendication 1, dans lequel ledit composé est de la N-(9-Methoxynonyl) déoxynojirimycine ou un de ses sels pharmaceutiquement acceptable.

9. Le composé selon la revendication 1, dans lequel R est

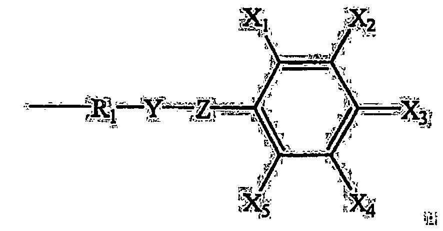

10. Le composé selon la revendication 9, dans lequel X₁ est NO₂ et X₃ est N₃.

11. Le composé selon la revendication 9, dans lequel X₂, X₄ et X₅ sont chacun un hydrogène.

12. Le composé selon la revendication 1, dans lequel ledit composé est de la N-(N- {4'-azido-2'-nitroplaenyl}-6-aminohexyl) déoxynojirimycine ou un de ses sels pharmaceutiquement acceptable.

13. Le composé selon la revendication 1, dans lequel le sujet est un mammifère, tel qu'un être humain.

14. Le composé selon la revendication 1, dans lequel le virus est choisi parmi le virus Ippy ; le virus de Lassa ; le virus de la chorioméningite lymphocytaire ; le virus Mobala ; le virus Mopeia ; le virus Amapari ; le virus Flexal ; le virus Guanarito ; le virus Junin ; le virus Latino ; le virus Machupo ; le viru Oliveros ; le virus Paraná; le virus Pichinde ; le virus Pirital ; le virus Sabiá; le virus Tacaribe ; le virus Tamiami ; le virus Whitewater Arroyo ; et le virus Chapare.

15. Le composé selon la revendication 1, dans lequel la maladie ou la condition est choisie parmi la chorioméningite lymphocytaire ; la fièvre de Lassa ; la fièvre hémorragique d'Argentine ; la fièvre hémorragique de Bolivie ; la fièvre hémorragique du Brésil ; la fièvre Tacaribe ; la fièvre hémorragique du Venezuela ; le syndrome grippal associé au virus Flexal ; et la fièvre hémorragique associée au virus Whitewater Arroyo.
